# EUROPÄISCHE PATENTSCHRIFT

(11) **EP 2 826 445 B1**
(45) Veröffentlichungstag und Bekanntmachung des Hinweises auf die Patenterteilung: **15.03.2017**
(21) Anmeldenummer: 14174580.2
(22) Anmeldetag: 26.06.2014
(51) Int. Cl.: A61F 2/38, A61F 2/46, A61F 2/30

(54) **Kniespacersystem mit einstellbarem Abstandhalter**
Knee spacer system with adjustable spacer
Système d'espaceur de genou équipé d'un écarteur réglable

(30) Priorität: 15.07.2013 DE 102013213831
(43) Veröffentlichungstag der Anmeldung: 21.01.2015
(73) Patentinhaber: Heraeus Medical GmbH, 61273 Wehrheim (DE)
(72) Erfinder: Vogt, Sebastian, 99092 Erfurt (DE)
(74) Vertreter: Heraeus IP

(56) Entgegenhaltungen:
- WO-A1-2010/015877
- US-A1- 2006 111 790
- US-A1- 2013 073 049
- US-B1- 6 482 209

## Beschreibung

Die Erfindung betrifft einen Kniespacer zum zeitweisen Ersetzen eines künstlichen Kniegelenks. Beschrieben wird auch ein Verfahren zum Anpassen eines Kniespacers an eine Behandlungssituation, aufweisend eine Tibiakomponente und einen Abstandhalter, wobei die Tibiakomponente eine Lauffläche zum beweglichen Anlegen an eine Femurkomponente aufweist und der Abstandhalter eine Auflagefläche zur Auflage auf die Tibia aufweist. Desweiteren wird auch die Verwendung eines solchen Kniespacers als temporärer Platzhalter in einem Knie eines Patienten beschrieben.

Ferner wird ein artikulierendes Kniespacersystem beschrieben, das aus einer Tibiakomponente und einem Abstandhalter und bevorzugt auch aus einer Femurkomponente zusammengesetzt ist. Das Kniespacersystem ist als temporärer Platzhalter im Rahmen von zweizeitigen septischen Revisionen von Kniegelenkendoprothesen bestimmt.

Die Merkmale des Oberbegriffs des Anspruchs 1 sind aus dem Dokument US 2006/0111790 A1 bekannt.

Gelenkendoprothesen haben gegenwärtig eine Standzeit von mehreren Jahren, zum Beispiel bei zementierten Hüftgelenkprothesen im Durchschnitt größer als zehn bis fünfzehn Jahre. Es gibt jedoch unerwünschte Lockerungen der Gelenkendoprothesen, die vor dem Erreichen der üblichen Standzeiten auftreten. Es wird dabei die septische und die aseptische Lockerung unterschieden. Bei der aseptischen Lockerung lassen sich bisher keine mikrobiellen Keime nachweisen. Die Ursachen der aseptischen Lockerungen können vielfältig sein. Häufig sind aseptische Lockerungen auf Abrieb an den Gleitflächen der Gelenkendoprothesen zurückzuführen.

Bei der septischen Lockerung wird der Lockerungsprozess durch mikrobielle Keime hervorgerufen. Man unterscheidet dabei in Abhängigkeit vom zeitlichen Auftreten frühe und späte Infektionen. Die septische Lockerung ist eine sehr ernste Erkrankung für den Patienten, die zusätzlich bei der Behandlung mit sehr hohen Kosten verbunden ist. Sowohl bei der aseptischen als auch der septischen Lockerung wird üblicherweise eine Revision vorgenommen. Man unterscheidet einzeitige und zweizeitige Revisionen. Bei septischen Lockerungen werden sehr häufig zweizeitige Revisionen vorgenommen.

Bei der zweizeitigen Revision wird in einer ersten Operation (OP) die infizierte Gelenkendoprothese entfernt, es erfolgt ein Debridement (ein Entfernen des infizierten Gewebes) und anschließend wird ein temporärer Platzhalter, ein so genannter Spacer, eingesetzt. Dieser Spacer füllt für einige Wochen den Raum der zuvor revidierten Endoprothese aus, bis die vorliegende Infektion abgeklungen ist. Diese Platzhalterfunktion ist sehr wichtig, um eine Atrophie der Muskulatur in dieser Zeit wirksam zu verhindern und um eine Stabilisierung der Resektionssituation zu erreichen.

Man unterscheidet nichtartikulierende und artikulierende Spacer. Artikulierende Spacer bilden die Gelenkfunktion nach und erlauben eine gewisse Beweglichkeit der betroffenen Gliedmaßen. Es ist dadurch möglich, die Patienten frühzeitig zu mobilisieren. Artikulierende Spacer werden daher derzeit besonders gerne eingesetzt. Der Spacer wird in einer zweiten OP entfernt, es wird nochmals debridiert und dann wird eine zementierte oder auch zementfreie Revisions-Gelenkendoprothese implantiert.

Die Verwendung von Spacern geht ursprünglich auf Hovelius und Josefsson zurück (Hovelius L, Josefsson G (1979), "An alternative method for exchange Operation of infected arthroplasty", Acta Orthop. Scand. 50: 93-96). Weitere frühe Arbeiten zu Spacern stammen von Younger (Younger AS, Duncan CP, Masri BA, McGraw RW (1997), "The outcome of two-stage arthroplasty using a custom-made interval spacer to treat the infected hip", J. Arthroplasty 12: 615-623), Jones (Jones WA, Wroblewski BM (1989), "Salvage of failed total knee arthroplasty: the 'beefburger' procedure", J. Bone Joint Surg. Br. 71: 856-857.) und Cohen (Cohen JC, Hozack WJ, Cuckler JM, Booth RE Jr (1988), "Two-stage reimplantation of septic total knee arthroplasty, Report of three cases using an antibiotic-PMMA spacer block", J. Arthroplasty 3: 369-377). Von McPherson stammt die Konzeption, dass Spacer ausschließlich aus Knochenzement hergestellt werden können (McPherson EJ, Lewonowski K, Dorr LD (1995), "Techniques in arthroplasty. Use of an articulated PMMA spacer in the infected total knee arthroplasty", J. Arthroplasty 10: 87-89).

Es gibt auf dem Markt mit Antibiotika ausgerüstete Kniespacer zum temporären Ersatz von Kniegelenkendoprothesen. Kniespacer sind im Allgemeinen zweiteilig und bestehen aus einer Tibiakomponente und einer Femurkomponente. Ein dafür typischer Spacer beziehungsweise ein dafür typisches Spacersystem ist aus der EP 1 274 374 A1 bekannt. Im Fall der Kniespacer müssen zusätzlich sowohl die Tibiakomponente als auch die Femurkomponente mit Polymethylmethacrylat-Knochenzement an der proximalen Tibia und am distalen Femur verankert werden. Dabei haftet der Polymethylmethacrylat-Knochenzement (PMMA-Knochenzement) an der Oberfläche der Kniespacerkomponenten. Die Oberfläche der Spacerkomponenten, die im Allgemeinen aus ausgehärtetem Antibiotika-dotiertem Polymethylmethacrylat-Knochenzement bestehen, wird durch das Methylmethacrylat des Polymethylmethacrylat-Knochenzements mehr oder weniger angelöst. Dadurch kommt es zum Verbund mit dem aushärtenden Polymethylmethacrylat-Knochenzement. Darauf beruht im Wesentlichen die Haftung des Polymethylmethacrylat-Knochenzements auf der Oberfläche der Spacerkomponenten.

Spacer, also auch Kniespacer, werden häufig während der Operation vom Arzt selbst per Hand aus Zementteig geformt oder unter Verwendung von elastischen Silikonformen selbst gegossen. Alternativ dazu gibt es seit mehreren Jahren auch industriell hergestellte Spacer, die in verschiedenen Standard-Größen angeboten werden. Diese vorgefertigten Spacer können vom Arzt ohne größere Vorbereitungsarbeiten direkt implantiert werden, wobei die Spacer üblicherweise mit Polymethylmethacrylat-Knochenzement am Knochengewebe fixiert wird. Der Vorteil der industriell hergestellten Spacer besteht darin, dass die Oberflächenqualität der Laufflächen (häufig auch als Gleitflächen bezeichnet) der vorgefertigten Spacer im Allgemeinen deutlich besser ist als die der intra-operativ gefertigten Spacer. Weiterhin kann durch die Verwendung von vorgefertigten Spacern wertvolle OP-Zeit gespart werden und der gesamte OP-Ablauf ist deutlich vereinfacht, weil der zeitaufwendige und arbeitsaufwendige Schritt der Herstellung der Spacer entfällt.

Bei septischen Revisionen von Kniegelenkendoprothesen tritt infolge des radikalen Debridements häufig ein deutlicher Verlust von Knochengewebe ein. Bei massivem Knochenverlust an der proximalen Tibia im Rahmen der zweizeitigen Revision können die Femurkomponente und die Tibiakomponente von artikulierenden Spacern die Gelenkfunktion temporär nur ersetzen, wenn an der Unterseite des Tibiaplateaus das fehlende Knochengewebe durch zum Teil mehrere Millimeter dicke Knochenzementschichten ersetzt wird, so dass annähernd die ursprüngliche Höhe des Tibiaplateaus erreicht wird, damit eine Artikulation der Femurkomponente mit der Tibiakomponente entsprechend der Anatomie des Knies des Patienten möglich ist. Diese Höheneinstellung ist für den Chirurgen schwierig, weil während der Verarbeitungsphase und auch noch zu Beginn der Aushärtungsphase des Knochenzements, der zur Fixierung der Tibiakomponente verwendet wird, eine plastische Verformung des Zementteigs möglich ist, wodurch sich der Abstand der Tibiakomponente von der proximalen Tibia unerwünscht verändern kann.

Die Aufgabe der Erfindung besteht also darin, die Nachteile des Stands der Technik zu überwinden. Insbesondere soll ein stabiler Kniespacer bereitgestellt werden, mit dem eine robuste und an die Behandlungssituation anpassbare Verbindung zur Tibia herstellbar ist. Bei der Behandlungssituation soll insbesondere ein Verlust des Knochengewebes durch vorherige Debridements ausgeglichen werden können. Mit der Erfindung soll ein Kniespacer beziehungsweise eine Tibiakomponente, die vorliegend auch ohne die zugehörige Femurkomponente bereits als Kniespacer bezeichnet wird, bereitstellen, mit der zuverlässig die anatomische Geometrie eines Kniegelenks eines Patienten durch den Spacer wiederhergestellt werden kann. Gleichzeitig soll der Kniespacer kostengünstig in der Fertigung sein. Der Erfindung liegt also die Aufgabe zu Grunde, ein Kniespacersystem zu entwickeln, das es ermöglicht, den Abstand zwischen der proximalen Tibia und der Tibiakomponente (beziehungsweise genauer der Lauffläche der Tibiakomponente) sicher einzustellen, ohne das eine Veränderung des Abstands während der Fixierung der Tibiakomponente mit Polymethylmethacrylat-Knochenzement eintreten kann.

Die Aufgaben der Erfindung werden durch einen Kniespacer gemäss den Merkmalen des Anspruchs 1 gelöst.

Beim Befestigen des Kniespacers an der Tibia des Patienten wird zwischen der Auflagefläche und dem Tibiaplateau eine Knochenzementschicht angeordnet, die nach dem Aushärten des Knochenzements den Abstandhalter an der Tibia fixiert. Der Abstandhalter liegt im Sinne der vorliegenden Erfindung also nicht unmittelbar auf der Tibia beziehungsweise dem Tibiaplateau auf, sondern es befindet sich zwischen dem Abstandhalter und dem Tibiaplateau eine Schicht Knochenzement. Diese Schicht kann zur Feinjustierung des Abstands der Lauffläche vom Tibiaplateau durch den Arzt verwendet werden.

Beschrieben wird ferner ein artikulierendes Kniespacersystem, das aus einer Tibiakomponente und einem Abstandhalter und bevorzugt auch aus einer Femurkomponente zusammengesetzt ist, wobei der Abstand zwischen der proximalen Seite (der Lauffläche) der Tibiakomponente und der distalen Seite (der Auflagefläche) des Abstandhalters eingestellt werden kann.

Mit dem beim Patienten eingesetzten Zustand ist die Anordnung gemeint, bei der die Komponenten des Kniespacers im Knie des Patienten einzuzementieren sind beziehungsweise einzementiert werden sollen, um ein funktionstüchtiges, das heißt bewegliches temporäres Implantat zu bilden.

Bei erfindungsgemäßen Kniespacern ist bevorzugt vorgesehen, dass an der der Lauffläche gegenüberliegenden Seite der Tibiakomponente ein Schaft mit einem Außengewinde angeordnet ist, auf den der Abstandhalter aufweisend zumindest eine Durchführung mit einem zum Außengewinde des Schafts passenden Innengewinde aufgeschraubt ist oder aufschraubbar ist, so dass der Abstand zwischen der Lauffläche und der Auflagefläche durch ein Schrauben des Abstandhalters auf den Schaft einstellbar ist.

Als Alternative zu dieser Ausführungsform könnte zum Einstellen des Abstands zwischen der Lauffläche und der Auflagefläche auch vorgesehen sein, dass der Schaft entlang des Abstands von der Lauffläche mehrere Ausnehmungen aufweist, wobei der Abstandhalter mit einem Bolzen oder einem anderen Arretierungsmittel, das in die Ausnehmungen greift, in unterschiedlichen Abständen befestigt werden kann. Erfindungsgemäß ist die Variante mit einem Außengewinde jedoch besonders bevorzugt, da der Abstandhalter so auf einfach und kostengünstig zu realisierende Weise mit sehr vielen unterschiedlichen Abständen zur Lauffläche eingestellt werden kann.

Des Weiteren kann vorgesehen sein, dass der Kniespacer eine Femurkomponente mit einer Lauffläche aufweist, wobei die Femurkomponente und die Tibiakomponente als separate und, im beim Patienten eingesetzten Zustand, gegeneinander bewegliche Komponenten vorliegen, wobei die Tibiakomponente und die Femurkomponente im beim Patienten eingesetzten Zustand über deren Laufflächen beweglich aneinander anlegbar sind.

Es ist also erfindungsgemäß bevorzugt, wenn mit der Tibiakomponente und dem Abstandhalter gleich eine passende Femurkomponente als Set mitgeliefert wird. Dies hat den Vorteil, dass die beiden Komponenten gleich aufeinander abgestimmt sind und so zueinander passende Laufflächen garantiert werden können.

Mit einer Weiterentwicklung der Erfindung wird vorgeschlagen, dass der Abstandhalter eine Scheibe, bevorzugt eine planparallele Scheibe aufweist, wobei eine Seite der Scheibe die Auflagefläche bildet.

Besonders bevorzugt ist der Abstandhalter eine solche Scheibe mit einer Durchbrechung, wobei in der ein Innengewinde angeordnet ist, so dass die planparallele Scheibe auf den Schaft mit dem Außengewinde aufgeschraubt werden kann.

Eine planparallele Scheibe ist besonders einfach zu fertigen, so dass diese Ausführungsform besonders kostengünstig zu realisieren ist.

Erfindungsgemäss sind in der Auflagefläche des Abstandhalters eine oder mehrere Durchbrechungen vorgesehen, durch die sich ein Knochenzement hindurch erstrecken kann, mit dem der Abstandhalter auf der Tibia befestigt wird oder befestigt ist.

Hierdurch können der Abstandhalter und damit der Kniespacer fester mit der Tibia verbunden werden, da der Knochenzement zur Befestigung des Abstandhalters durch die Durchbrechung oder die Durchbrechungen hindurch fließt und hindurch greift.

Gemäß einer bevorzugten Ausführungsform kann vorgesehen sein, dass der Kniespacer aus einem biokompatiblen Material besteht, bevorzugt aus ausgehärteten Polymethylmethacrylat-Knochenzement besteht, der mindestens ein Antibiotikum und/oder ein Antiseptikum enthält.

Diese Materialien sind besonders gut zur Verwendung im menschlichen Körper geeignet. Der Knochenzement, der zur Befestigung der Komponente beziehungsweise der Komponenten des Kniespacers verwendet wird, ist bevorzugt der gleiche, aus dem auch die Tibiakomponente und der Abstandhalter und gegebenenfalls die Femurkomponente gefertigt sind. Es kann für bestimmte Anwendungen jedoch auch vorgesehen sein, dass der Polymethylmethacrylat-Knochenzement (PMMA-Knochenzement), aus dem der Kniespacer besteht, eine andere Zusammensetzung, insbesondere andere Antibiotika und/oder Antiseptika aufweist.

Zur Umsetzung erfindungsgemäßer Kniespacer kann vorgesehen sein, dass die Tibiakomponente und gegebenenfalls die Femurkomponente mit zumindest einem Kunststoff und/oder Metall aufgebaut sind, bevorzugt aus Kunststoff und/oder Metall bestehen, besonders bevorzugt aus Polymethylmethacrylat, ganz besonders bevorzugt aus mit Antibiotika dotiertem ausgehärtetem Polymethylmethacrylat-Knochenzement.

Diese Materialien eignen sich zur Herstellung erfindungsgemäßer Kniespacer besonders gut.

Ferner kann vorgesehen sein, dass die Tibiakomponente einen Schaft aufweist, der sich von einer zentralen Position der der Lauffläche gegenüberliegenden Seite der Tibiakomponente mit einem Winkel zwischen 85° und 90° zur Lauffläche aus erstreckt, bevorzugt senkrecht zur Lauffläche aus erstreckt. Bevorzugt ist der Schaft zur Verankerung in einer Ausnehmung in der Tibia vorgesehen. Ebenfalls bevorzugt ist der Schaft zylindrisch und weist ein Außengewinde auf.

Der Schaft dient der Verankerung in der Tibia. Dadurch ist eine besonders stabile Verbindung zur Tibia erreichbar. Zudem kann über das Außengewinde eine variable und stabile Einstellung des Abstands der Auflagefläche des Abstandhalters und der Lauffläche der Tibiakomponente erfolgen.

Es kann auch vorgesehen sein, dass auf der Auflagefläche der Tibiakomponente mehrere Abstandhalter angeordnet sind, wobei die Abstandhalter bevorzugt von der Auflagefläche aus zumindest bereichsweise zusammenlaufend sind, und besonders bevorzugt der Schaft zumindest viermal so hoch ist wie die Abstandhalter der Auflagefläche der Tibiakomponente.

Die Abstandhalter dienen dazu, dass eine ausreichende Menge und eine ausreichende Schichtdicke des Knochenzements zur Verbindung der Komponenten mit dem Knochen verbleiben und so eine stabile Verbindung der Komponenten mit den Knochen erzeugt wird.

Beschreiben wird ferner ein Verfahren zum Anpassen eines Kniespacers an eine Behandlungssituation, insbesondere eines solchen Kniespacers, aufweisend eine Tibiakomponente und einen Abstandhalter, wobei die Tibiakomponente eine Lauffläche zum beweglichen Anlegen an eine Femurkomponente aufweist und der Abstandhalter eine Auflagefläche zur Auflage auf die Tibia aufweist, wobei bei dem Verfahren der Abstand zwischen der Auflagefläche und der Lauffläche eingestellt wird.

Dabei kann vorgesehen sein, dass der Abstand zwischen der Auflagefläche und der Lauffläche durch Drehen des Abstandhalters auf einem Schaft mit einem Außengewinde eingestellt wird, wobei der Schaft auf der der Lauffläche gegenüberliegenden Seite der Tibiakomponente befestigt ist und der Abstandhalter wenigstens eine Durchführung mit einem Innengewinde aufweist, so dass durch das Drehen des Abstandhalters der Abstandhalter auf dem Schaft geschraubt wird.

Schließlich wird die Verwendung eines solchen Kniespacers als temporärer Platzhalter in einem Knie eines Patienten beschrieben.

Der Erfindung liegt die Erkenntnis zugrunde, dass es durch die Verwendung eines variablen Abstandhalters gelingt, einen Kniespacer beziehungsweise eine Tibiakomponente für einen Kniespacer bereitzustellen, der stabil auf ein Tibiaplateau aufgesetzt und dort befestigt werden kann, und mit dem gleichzeitig eine variable Länge eines fehlenden Tibiastücks ersetzt werden kann, das zuvor entfernt wurde. Der Kniespacer beziehungsweise die Tibiakomponente kann also zum temporären Ersatz eines Kniegelenks verwendet werden unabhängig davon, wie stark die Tibia bis zum so erzeugten Tibiaplateau abgetragen wurde. Gleichzeitig ist die Tibiakomponente sehr stabil auf dem Tibiaplateau aufsetzbar, ohne dass befürchtet werden muss, dass diese beim Aushärten des Knochenzements, mit dem die Tibiakomponente befestigt wird, verkippt oder der Knochenzement herausgepresst wird. Die Tibiakomponente ist also verkippungssicher und einfach zu positionieren.

Ein Kniespacersystem ist aus wenigstens einer Femurkomponente, einer Tibiakomponente und einem Abstandhalter zusammengesetzt.

Das Kniespacersystem ist bevorzugt dadurch charakterisiert, dass
a) an der distalen Seite der Tibiakomponente ein Schaft angeordnet ist, der mindestens ein Außengewinde enthält, und
b) dass ein Abstandhalter, der eine Ausnehmung enthält, an deren Innenseite mindestens ein Innengewinde angeordnet ist, auf dem Schaft so drehbar angeordnet ist, dass der Abstand zwischen der distalen Seite der Tibiakomponente und der proximalen Seite Abstandhalters durch Drehung des Abstandhalters auf dem Schaft eingestellt werden kann.

Die Tibiakomponente von primären Kniegelenkendoprothesen besitzt an ihrer distalen Seite üblicherweise einen Schaft, der circa zwei bis fünf Zentimeter lang ist und für die Verankerung der Tibiakomponente in der proximalen Tibia benötigt wird. Das bedeutet, dass dadurch bei Revisionen nach erfolgter Entfernung der Tibiakomponente des Kniegelenks eine circa 2 bis 5 cm tiefe Kavität in der proximalen Tibia vorhanden ist. Dadurch kann an der Tibiakomponente des Kniespacersystems ebenfalls ein Schaft an der distalen Seite ausgebildet sein.

Erfindungsgemäß ist, dass der Abstandhalter als Scheibe ausgebildet ist, wobei die proximale und die distale Seite des Abstandhalters bevorzugt plan sind, besonders bevorzugt planparallel sind.

Der Abstandhalter enthält bevorzugt eine oder mehrere Durchbrechungen, welche die distale und die proximale Seite des Abstandhalters verbinden. Das bedeutet, dass der medizinische Anwender problemlos Polymethylmethacrylat-Knochenzement durch diese Durchbrechungen in den Raum zwischen dem Abstandhalter und der distalen Seite der Tibiakomponente einbringen kann, so dass dieser Raum komplett gefüllt ist. Dadurch werden ein sicherer Verbund und damit eine Kraftübertragung zwischen der Tibiakomponente und dem Abstandhalter gewährleistet. Die Tibiakomponete wird mit dem Abstandhalter verbunden, so dass eine einheitliche Komponente gebildet wird. An der distalen Seite des Abstandhalters wird ebenfalls Polymethylmethacrylat-Knochenzement aufgebracht und mit dieser Zementschicht wird die aus der Tibiakomponente und dem Abstandhalter gebildete verbundene Komponente an der proximalen Tibia fixiert.

Das Kniespacersystem besteht aus einem biokompatiblen Material. Das Kniespacersystem besteht bevorzugt aus ausgehärtetem Polymethylmethacrylat-Knochenzement, der mindestens ein Antibiotikum und/oder ein Antiseptikum enthält. Als Antibiotika kommen dabei alle in der Orthopädie üblichen Antibiotika in Betracht. Besonders geeignet sind Gentamcin, Tobramycin, Clindamycin, Vancomycin, Daptomycin und Fosfomycin. Ebenfalls geeignet sind alle sonstigen Antiinfektiva. Es ist auch möglich, dass Antiseptika im Polymethylmethacrylat-Knochenzement enthalten sind. Hierbei kommen besonders Polyhexanid, Octenidin und Wasserstoffperoxid abspaltende Substanzen, wie Calciumperoxid, Harnstoff-Wasserstoffperoxid-Addukt in Betracht.

Ein Verfahren ist dadurch charakterisiert, dass der Abstand zwischen der distalen Seite der Tibiakomponente und der proximalen Seite des Abstandhalters durch Drehung des Abstandhalters auf dem Gewinde des Schaftes eingestellt wird.

Der erfindungsgemäße Kniespacer wird als temporärer Platzhalter im Rahmen von zweizeitigen septischen Revisionen von Kniegelenkendoprothesen verwendet.

Im Folgenden werden Ausführungsbeispiele der Erfindung anhand von fünf schematisch dargestellten Figuren erläutert, ohne jedoch dabei die Erfindung zu beschränken. Dabei zeigt:
- Figur 1:: eine schematische Seitenansicht eines erfindungsgemäßen Kniespacers;
- Figur 2:: eine schematische Seitenansicht eines erfindungsgemäßen Kniespacers, bei dem der Abstandhalter auf die Tibiakomponente geschraubt ist;
- Figur 3:: eine schematische Aufsicht auf die Lauffläche einer Tibiakomponente für einen erfindungsgemäßen Kniespacer;
- Figur 4:: eine schematische Aufsicht auf einen Abstandhalter für einen erfindungsgemäßen Kniespacer; und
- Figur 5:: eine schematische teilweise Querschnittansicht eines erfindungsgemäßen Kniespacers aufweisend eine Tibiakomponente, eine Femurkomponente und einen Abstandhalter.

In den Figuren werden teilweise für gleiche oder gleichartige Bauteile die gleichen Bezugszeichen verwendet.

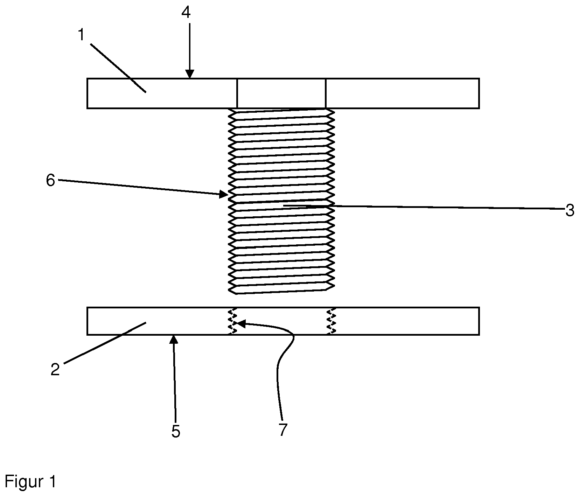

Figur 1 zeigt eine schematische Seitenansicht eines erfindungsgemäßen Kniespacers. Der Kniespacer weist eine Tibiakomponente 1 und eine Abstandhalterplatte 2 auf, die aus ausgehärtetem PMMA-Knochenzement enthaltend wenigstens ein Antibiotikum und/oder Antiseptikum gefertigt sind. Die Tibiakomponente 1 besteht aus einer anatomisch passenden Platte und einem zylindrischen Schaft 3, der auf der Unterseite der Platte (in Figur 1 unten) angeordnet ist. Auf der Oberseite der Platte der Tibiakomponente 1 ist eine Lauffläche 4 vorgesehen, die als Gleitfläche für eine Lauffläche einer Femurkomponente (in Figur 1 nicht gezeigt) vorgesehen ist.

Der Abstandhalter 2 ist durch eine planparallele Platte gebildet, auf deren Unterseite die Auflagefläche 5 zum Auflegen auf das Tibiaplateau vorgesehen ist. Der Schaft 3 weist ein Außengewinde 6 auf, das zu einem Innengewinde 7 in einer Durchführung des Abstandhalters 2 passt (in Figur 1 durch eine gepunktete Linie angedeutet, da das Innengewinde 7 eigentlich von der Seite aus nicht sichtbar ist). Der Abstandhalter 2 kann so auf den Schaft 3 aufgeschraubt werden.

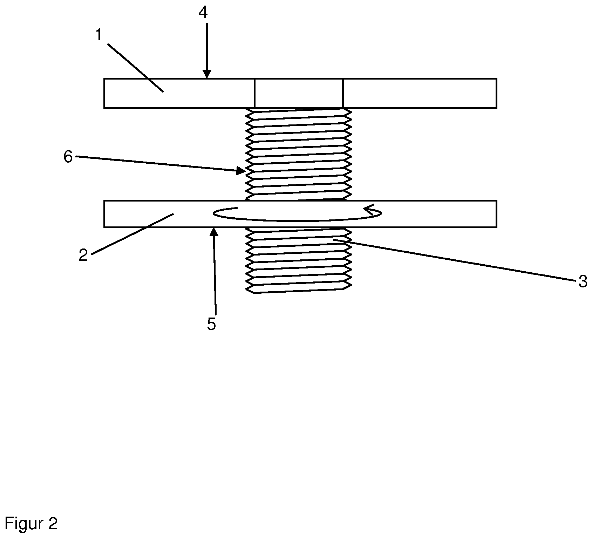

Figur 2 zeigt eine schematische Seitenansicht des Kniespacers nach Figur 1, bei dem der Abstandhalter 2 auf die Tibiakomponente 1 beziehungsweise den Schaft 3 geschraubt ist. Durch Drehen des Abstandhalters 2 um die Achse des Schafts 3 kann der Abstand der Auflagefläche 5 des Abstandhalters 2 zur Lauffläche 4 der Tibiakomponente 1 eingestellt werden. Bei einer Drehung, wie diese in Figur 2 durch den Pfeil angedeutet ist, wird der Abstandhalter 2 in Richtung der Platte der Tibiakomponente 1 versetzt und dadurch der Abstand der Auflagefläche 5 zur Lauffläche 4 verkürzt. Theoretisch könnte auch ein Linksgewinde am Schaft 3 angeordnet sein und sich dadurch der Drehsinn umkehren.

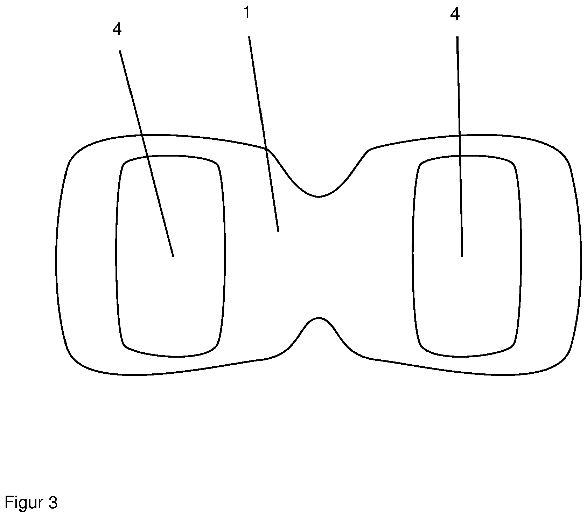

Figur 3 zeigt eine schematische Aufsicht auf die Lauffläche 4 einer Tibiakomponente 1 für einen erfindungsgemäßen Kniespacer. Die Lauffläche 4 ist dazu mit glatter Oberfläche gestaltet, so dass eine Lauffläche einer Femurkomponente gut über die Lauffläche 4 der Tibiakomponente 1 gleiten kann.

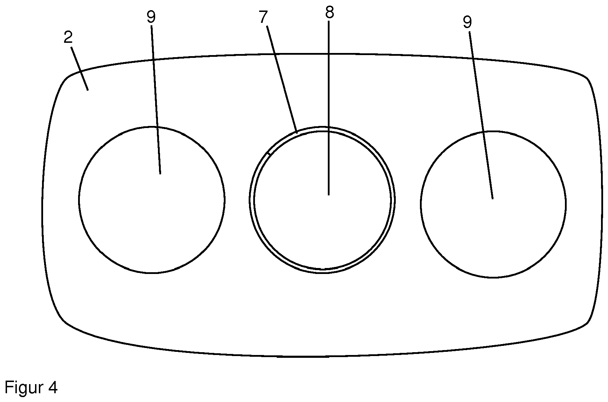

Figur 4 zeigt eine schematische Aufsicht auf einen Abstandhalter 2 für einen erfindungsgemäßen Kniespacer. In dem Abstandhalter 2 ist eine zentrale Durchführung 8 vorgesehen, in der das Innengewinde 7 angeordnet ist. Der Abstandhalter 2, der als planparallele Platte ausgebildet ist, umfasst des Weiteren zwei weitere Bohrungen 9, die auf beiden Seiten der Durchführung 8 mit dem Innengewinde 7 angeordnet sind und die sich vollständig durch den Abstandhalter 2 erstrecken. Beim Einsetzen des Abstandhalters 2 in ein Knie eines Patienten, wird der Abstandhalter 2 mit Knochenzement auf einem künstlich erzeugten Tibiaplateau (nicht gezeigt) befestigt. Der Knochenzement kann dabei durch die Bohrungen 9 fließen und verankert nach dem Aushärten des Knochenzements den Abstandhalter 2 an der Tibia.

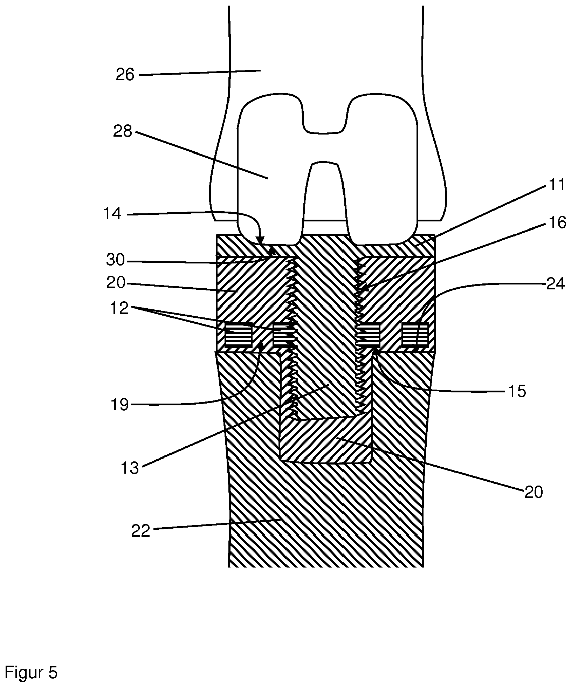

Figur 5 zeigt eine schematische teilweise Querschnittansicht eines erfindungsgemäßen Kniespacers beziehungsweise eines Kniespacersystems aufweisend eine Tibiakomponente 11, eine Femurkomponente 28 und einen Abstandhalter 12. Der plattenförmige Abstandhalter 12, der auf die Tibiakomponente 11 aufgeschraubt ist, weist an der Unterseite (in Figur 5 unten) eine Auflagefläche 15 auf, die auf einem Tibiaplateau 24 einer Tibia 22 (eines Schienbeinknochens) aufgelegt ist und mit Knochenzement 20 befestigt beziehungsweise eingegossen ist.

Der gesamte zur Tibia 22 gehörende Teil des Kniespacers sowie die Tibia 22 selbst sind in einer Querschnittdarstellung gezeigt, wobei die schraffierten Oberflächen geschnitten sind. Der Schnitt erfolgt in einer Ebene senkrecht zur Sagittalebene beziehungsweise parallel zur oder in der Frontalebene. Die Querschnittansicht ermöglicht die Darstellung des inneren Aufbaus der Tibiakomponente 11 und deren Verankerung. Die Femurkomponente 28, die an einem Femur 26 des Patienten mit Knochenzement befestigt ist, ist dagegen in Seitenansicht dargestellt.

Die Femurkomponente 28 weist eine abgerundete Lauffläche 30 auf, die auf der Lauffläche 14 der Tibiakomponente 11 gleiten beziehungsweise abrollen kann. Dadurch wird ein artikulierender zweiteiliger Kniespacer bereitgestellt. In der Tibia 22 ist eine Ausnehmung zur Aufnahme des Schafts 13 vorgesehen. Je nachdem, wie viel Knochen vom Tibiaplateau 24 der Tibia 22 entfernt wurde, kann die ursprüngliche Lage der Lauffläche 14 des Knies dadurch wieder eingestellt werden, indem der Abstandhalter 12 bis zu einer gewissen Höhe auf ein Außengewinde 16 des Schafts 13 aufgeschraubt wird. Dazu weist der Abstandhalter 12 eine Durchführung mit einem passenden Innengewinde auf. Dadurch ist ohne großen Aufwand eine relativ genaue Einstellung des Abstands zwischen der Lauffläche 14 der Tibiakomponente 11 und der Auflagefläche 15 des Abstandhalters 12 und damit eine relativ genaue Einstellung der Lage des künstlichen Kniegelenks möglich.

In dem kreisrunden Abstandhalter 12 sind mehrere Bohrungen 19 vorgesehen, durch die sich der Knochenzement 20 erstreckt und damit den Abstandhalter 12 und die Tibiakomponente 11 stabil mit der Tibia 22 verbindet beziehungsweise verankert.

Bei einer Revision eines künstlichen Kniegelenks, kann für die Operation nach dem Debridieren des Tibia-Knochens 22 die Lage des Gelenks des artikulierenden Kniespacers durch Schrauben beziehungsweise Drehen des Abstandhalters 12 auf dem Schaft 13 eingestellt werden. Wenn der dazu notwendige Abstand zwischen der Auflagefläche 15 des Abstandhalters 12 zur Lauffläche 14 der Tibiakomponente 11 eingestellt ist, wird die Tibiakomponente 11 mit dem Abstandhalter 12 mit Hilfe des PMMA-Knochenzements 20 auf das Tibiaplateau 24 zementiert und die Zwischenräume zwischen der Tibiakomponente 11 und dem Abstandhalter 12 sowie die Bohrungen 19 werden mit dem gleichen PMMA-Knochenzement 20 aufgefüllt. Ebenso wird die Femurkomponente 28 mit dem gleichen PMMA-Knochenzement auf dem Femur 26 befestigt.

Der Kniespacer ist dann eingesetzt und funktionsfertig. Wenn die Teile 11, 12, 28 des Kniespacers und der Knochenzement 20 zumindest ein Antibiotikum und/oder Antiseptikum enthalten, kann der Kniespacer zur Bekämpfung eines Infektionsherds im Knie verwendet werden.

Anstatt scharfer Kanten und Ecken sind für Kniespacer beziehungsweise deren Komponenten 1, 2, 18 abgerundete Ecken und Kanten bevorzugt.

### Bezugszeichenliste

- 1: Tibiakomponente
- 2: Abstandhalterplatte
- 3: Schaft
- 4: Lauffläche
- 5: Auflagefläche
- 6: Außengewinde
- 7: Innengewinde
- 8: Durchführung
- 9: Bohrung
- 11: Tibiakomponente
- 12: Abstandhalterplatte
- 13: Schaft
- 14: Lauffläche
- 15: Auflagefläche
- 16: Außengewinde
- 19: Bohrung
- 20: PMMA-Knochenzement
- 22: Tibia
- 24: Tibiaplateau
- 26: Femur
- 28: Femurkomponente
- 30: Lauffläche

## Patentansprüche

1. Kniespacer zum zeitweisen Ersetzen eines künstlichen Kniegelenks, wobei der Kniespacer eine Tibiakomponente (1, 11) und einen Abstandhalter (2, 12) aufweist, wobei die Tibiakomponente (1, 11) eine Lauffläche (4, 14) aufweist, über die die Tibiakomponente (1, 11) beweglich an eine Femurkomponente (28) im beim Patienten eingesetzten Zustand anlegbar ist, und wobei der Abstandhalter (2, 12) eine Auflagefläche (5, 15) zur Auflage auf die Tibia (22) aufweist und die Auflagefläche (5, 15) mit einem variablen Abstand zur Lauffläche (4, 14) der Tibiakomponente (1, 11) einstellbar ist, **dadurch gekennzeichnet, dass**
in der Auflagefläche (5, 15) des Abstandhalters (2, 12) eine oder mehrere Durchbrechungen (9, 19) vorgesehen sind, durch die sich ein Knochenzement (20) hindurch erstrecken kann, mit dem der Abstandhalter (2, 12) auf der Tibia (22) befestigt wird oder befestigt ist.

2. Kniespacer nach Anspruch 1, **dadurch gekennzeichnet, dass**
an der, der Lauffläche (4, 14) gegenüberliegenden Seite der Tibiakomponente (1, 11) ein Schaft (3, 13) mit einem Außengewinde (6, 16) angeordnet ist, auf den der Abstandhalter (2, 12) aufweisend zumindest eine Durchführung (8) mit einem zum Außengewinde (6, 16) des Schafts (3, 13) passenden Innengewinde (7) aufgeschraubt ist oder aufschraubbar ist, so dass der Abstand zwischen der Lauffläche (4, 14) und der Auflagefläche (5, 15) durch ein Schrauben des Abstandhalters (2, 12) auf den Schaft (3, 13) einstellbar ist.

3. Kniespacer nach Anspruch 1 oder 2, **dadurch gekennzeichnet, dass**
der Kniespacer eine Femurkomponente (28) mit einer Lauffläche (30) aufweist, wobei die Femurkomponente (28) und die Tibiakomponente (1, 11) als separate und, im beim Patienten eingesetzten Zustand, gegeneinander bewegliche Komponenten (1, 11, 28) vorliegen, wobei die Tibiakomponente (1, 11) und die Femurkomponente (28) im beim Patienten eingesetzten Zustand über deren Laufflächen (4, 14, 30) beweglich aneinander anlegbar sind.

4. Kniespacer nach einem der vorangehenden Ansprüche, **dadurch gekennzeichnet, dass**
der Abstandhalter (2, 12) eine Scheibe (2, 12), bevorzugt eine planparallele Scheibe (2, 12) aufweist, wobei eine Seite der Scheibe (2, 12) die Auflagefläche (5, 15) bildet.

5. Kniespacer nach einem der vorangehenden Ansprüche, **dadurch gekennzeichnet, dass**
der Kniespacer aus einem biokompatiblen Material besteht, bevorzugt aus ausgehärteten Polymethylmethacrylat-Knochenzement besteht, der mindestens ein Antibiotikum und/oder ein Antiseptikum enthält.

## Claims

1. Knee spacer for temporary replacement of an artificial knee joint, whereby the knee spacer comprises a tibial component (1, 11) and a spacer (2, 12), whereby the tibial component (1, 11) comprises a running surface (4, 14) by means of which the tibial component (1, 11) can be placed against a femoral component (28) in the patient-inserted state such as to be mobile, and whereby the spacer (2, 12) comprises a support surface (5, 15) for support on the tibia (22) and the support surface (5, 15) is adjustable such as to be at a variable distance from the running surface (4, 14) of the tibial component (1, 11), **characterised in that**
one or more perforations (9, 19) are provided in the support surface (5, 15) of the spacer (2, 12) through which a bone cement (20) can extend by means of which the spacer (2, 12) is to be or is attached on the tibia (22).

2. Knee spacer according to claim 1, **characterised in that**
the side of the tibial component (1, 11) opposite from the running surface (4, 14) has a shaft (3, 13) with an external thread (6, 16) arranged on it onto which the spacer (2, 12) comprising at least one perforation (8) with an internal thread (7) fitting the external thread (6, 16) of the shaft (3, 13) is or is to be screwed such that the distance between the running surface (4, 14) and the support surface (5, 15) can be adjusted by screwing the spacer (2, 12) onto the shaft (3, 13).

3. Knee spacer according to claim 1 or 2, **characterised in that**
the knee spacer comprises a femoral component (28) with a running surface (30), whereby the femoral component (28) and the tibial component (1, 11) are present as components (1, 11, 28) that are separate and, in the patient-inserted state, can be moved with respect to each other, whereby the tibial component (1, 11) and the femoral component (28), in the patient-inserted state, can be placed against each other such as to be mobile by means of their running surfaces (4, 14, 30).

4. Knee spacer according to any one of the preceding claims, **characterised in that**
the spacer (2, 12) comprises a disk (2, 12), preferably a planar-parallel disk (2, 12), whereby one side of the disk (2, 12) forms the support surface (5, 15).

5. Knee spacer according to any one of the preceding claims, **characterised in that**
the knee spacer consists of a biocompatible material, preferably consists of cured polymethylmethacrylate bone cement that contains at least one antibiotic and/or one anti-septic agent.

## Revendications

1. Espaceur de genou pour le remplacement temporaire d'une articulation de genou artificielle, dans lequel l'espaceur de genou présente un composant de tibia (1, 11) et un écarteur (2, 12), dans lequel le composant de tibia (1, 11) présente une surface de roulement (4, 14) au-dessus de laquelle le composant de tibia (1, 11) peut être appliqué de manière mobile sur un composant de fémur (28) dans l'état utilisé chez le patient, et dans lequel l'écarteur (2, 12) présente une surface d'appui (5, 15) pour l'appui sur le tibia (22) et la surface d'appui (5, 15) peut être réglée avec un écart variable par rapport à la surface de roulement (4, 14) du composant de tibia (1, 11), **caractérisé en ce que**
un ou plusieurs évidements (9, 19) à travers lesquels peut s'étendre un ciment osseux (20) avec lequel l'écarteur (2, 12) est fixé sur le tibia (22) sont prévus dans la surface d'appui (5, 15) de l'écarteur (2, 12).

2. Espaceur de genou selon la revendication 1, **caractérisé en ce que**
une tige (3, 13) avec un filetage externe (6, 16) sur laquelle l'écarteur (2, 12) présentant au moins un passage (8) est vissé ou peut être vissé avec un filetage interne (7) correspondant au filetage externe (6, 16) de la tige (3, 13), de sorte que l'écart entre la surface de roulement (4, 14) et la surface d'appui (5, 15) peut être réglé par un vissage de l'écarteur (2, 12) sur la tige (3, 13), est disposée sur le côté du composant de tibia (1, 11) opposé à la surface de roulement (4, 14).

3. Espaceur de genou selon la revendication 1 ou 2, **caractérisé en ce que**
l'espaceur de genou présente un composant de fémur (28) avec une surface de roulement (30), dans lequel le composant de fémur (28) et le composant de tibia (1, 11) sont présents en tant que composants séparés (1, 11, 28) et mobiles l'un contre l'autre dans l'état utilisé chez le patient, dans lequel le composant de tibia (1, 11) et le composant de fémur (28) peuvent être appliqués de manière mobile l'un sur l'autre au-dessus de leurs surfaces de roulement (4, 14, 30) dans l'état utilisé chez le patient.

4. Espaceur de genou selon une des revendications précédentes, **caractérisé en ce que**
l'écarteur (2, 12) présente un disque (2, 12), de préférence un disque parallélépipédique (2, 12), dans lequel un côté du disque (2, 12) forme la surface d'appui (5, 15).

5. Espaceur de genou selon une des revendications précédentes, **caractérisé en ce que**
l'espaceur de genou se compose d'un matériau biocompatible, se compose de préférence de ciment osseux de polyméthylméthacrylate durci qui contient au moins un antibiotique et/ou un antiseptique.
